Europäisches Patentamt

European Patent Office

Office européen des brevets

(11) Numéro de publication: **0 419 298 A1**

## DEMANDE DE BREVET EUROPEEN

(21) Numéro de dépôt: **90402115.1**

(22) Date de dépôt: **23.07.90**

(51) Int. Cl.⁵: **A61K 45/05, A61K 35/22**

(30) Priorité: **25.07.89 FR 8910010**

(43) Date de publication de la demande:
**27.03.91 Bulletin 91/13**

(84) Etats contractants désignés:
**AT BE CH DE DK ES FR GB GR IT LI LU NL SE**

(71) Demandeur: **INSTITUT PASTEUR**
**28, rue du Docteur Roux**
**F-75724 Paris Cédex 15(FR)**

(72) Inventeur: **Fauve, Robert**
**18, rue de la Justice**
**F-92310 Sevres(FR)**
Inventeur: **Fontan, Elizabeth**
**16, rue des Abondances**
**F-92100 Boulogne(FR)**

(74) Mandataire: **Le Guen, Gérard et al**
**CABINET LAVOIX 2, place d'Estienne d'Orves**
**F-75441 Paris Cédex 09(FR)**

(54) Protéines immunostimulantes, anticorps monoclonaux et polyclonaux correspondants, hybridomes correspondants et application de ces produits à des fins thérapeutiques et de diagnostic.

(57) L invention a pour objet une glycoprotéine immunostimulante ayant les caractéristiques suivantes :

a. elle a une masse moléculaire d'environ 43 kD
b. elle résiste à l'hydrolyse par la trypsine et par la pronase
c. elle présente un pourcentage en sucre d'environ 10%
d. elle peut être obtenue à partir d'urine humaine
e. elle comprend les 2 séquences peptidiques suivantes :
- peptide 1 : Met-Ala-Glu-Thr-Glu-Val-Pro-Phe-Leu-Arg-Asp-Asn - peptide 2 : Met-Ser-Leu-Val-Ile-Pro-Gly-Val.

EP 0 419 298 A1

La présente invention concerne des protéines humaines immunostimulantes, leur procédé d'obtention et leurs applications à des fins thérapeutiques et de diagnostic.

Il a été démontré chez des souris qu'une réaction inflammatoire induite par des substances non diffusibles, non biodégradables et non antigéniques, induit, à distance du site de la réaction inflammatoire, une augmentation de résistance envers divers agents pathogènes tels que bactéries, champignons, parasites et même envers des cellules malignes. Toujours chez la souris, il a été trouvé qu'une fraction protéique isolée du site de la réaction inflammatoire rendait compte, au moins partiellement de cette augmentation de résistance (FAUVE et al, in "Phagocytosis - past and future", M L. Karnovsky & L. Bolis, eds. 1982, Academic Press, New York, pp. 295-322). Il fut ensuite montré que l'activité immunostimulante de cette fraction protéique était entièrement représentée par une protéine. Cette dernière, " mouse granuloma protein" ou MGP (FONTAN et al., Proc. Natl. Acad. Sci. USA, 1983, 80 , 6395-6398) a un poids moléculaire de 56 kilodaltons (kD), un pHi de 5 et est capable, après injection à la souris (250 μg/kg) de protéger totalement celle-ci contre une infection létale à Listeria monocytogenes . De plus, cette protéine mise en présence de cellules péritonéales de souris, rend ces cellules cytostatiques envers les cellules de la tumeur de Lewis (FONTAN el al, Int. J. Cancer, 1988, 42 , 267-272).

Grâce à des anticorps anti-MGP il a été possible de détecter dans un sérum de malade, par test ELISA, une protéine présentant un ou des épitopes communs avec MGP. Cette protéine immunoréactive étant présente dans une fraction protéique de poids moléculaire légèrement inférieure à celui de l'albumine humaine, nous avons recherché cette fraction dans l'urine humaine Un test ELISA réalisé avec des anticorps anti-MGP étant positif, la purification de cette protéine puis sa caractérisation ont été entreprises

La présente invention a ainsi pour objet une glycoprotéine immunostimulante ayant les caractéristiques suivantes :

a. elle a une masse moléculaire d'environ 43 kD

b. elle résiste à l'hydrolyse par la trypsine et par la pronase

c. elle présente un pourcentage en sucre d'environ 10%

d. elle peut être obtenue à partir d'urine humaine.

La présente invention a également pour objet des peptides obtenus après hydrolyse par le bromure de cyanogène de la glycoprotéine immunostimulante purifiée selon l'invention. Ces peptides répondent aux formules :

- peptide 1 : Met-Ala-Glu-Thr-Glu-Val-Pro-Phe-Leu

Arg-Asp-Asn
- peptide 2 : Met-Ser-Leu-Val-Ile-Pro-Gly-Val

La présente invention a également pour objet la protéine native ayant une masse moléculaire d'environ 92 kD, qui présente une activité immunostimulante et qui donne la glycoprotéine selon l'invention par hydrolyse par la pronase.

La présente invention a également pour objet un procédé d'obtention de la protéine native selon l'invention et qui consiste essentiellement à séparer cette protéine de l'urine humaine.

Les étapes du procédé consistent à :
- collecter, conserver et traiter les urines,
- effectuer une chromatographie sur une colonne de DEAE Affigel$^R$ blue qui permet d'éliminer l'albumine et d'obenir la protéine immunostimulante avec un gradient de chlorure de sodium.

La glycoprotéine immunostimulante selon l'invention peut être obtenue à partir de la protéine native de masse moléculaire d'environ 92 kD par un procédé consistant à :
- hydrolyser la protéine native de masse moléculaire d'environ 92 kD par la pronase,
- effectuer une filtration sur tamis moléculaire, et
- éliminer l'$\alpha_1$-glycoprotéine acide par passage sur une colonne d'immunoaffinité.

La présente invention a également pour objet une composition thérapeutique comprenant à titre de principe actif les produits selon l'invention.

Les compositions thérapeutiques selon l'invention peuvent être administrées à l'homme ou aux animaux par voie topique, orale ou parentérale.

Elles peuvent être sous la forme de préparations solides, semi-solides ou liquides. Comme exemples, on peut citer les comprimés, les gélules, les suppositoires, les solutions ou suspensions injectables, les pommades, les collyres huileux ou aqueux, les collutoires, les solutions nasales et otologiques, ainsi que les formes retard.

Dans ces compositions le principe actif est généralement mélangé avec un ou plusieurs excipients pharmaceutiquement acceptables habituels bien connus de l'homme de l'art.

Les compositions thérapeutiques administrables par voie topique peuvent contenir notamment de 0,1 à 5% en poids du principe actif.

Les compositions thérapeutiques administrables par voie orale ou parentérale peuvent contenir notamment de 1% à 60% en poids de principe actif.

La quantité de principe actif administré dépend évidemment du patient qui est traité, de la voie d'administration et de la sévérité de la maladie. Cependant, pour l'administration par la voie parentérale, on peut administrer le principe actif à la dose de 50 à 500 microgrammes/kg. pour la voie orale on peut administrer environ de 0,5 à 5 mg/kg de principe actif.

Les compositions selon l'invention peuvent être utilisées pour stimuler la résistance contre un agent pathogène.

Les compositions selon l'invention peuvent être utilisées notamment dans le traitement des infections par Listeria monocytogenes , Mycobacterium tubertulosis , Pseudomonas aeruginosa et plus généralement contre des agents pathogènes capables de se multiplier à l'intérieur des cellules telles que les macrophages.

Elles peuvent être utilisées notamment dans des traitements post-opératoires pour des opérations à haut risque ainsi que dans des situations d'immunodépression (cancer, greffes, brûlures) où elles protègent les patients contre des infections à germes pathogènes fréquents dans ces situations, telles que les infections à Pseudomonas aeruginosa , Candida albicans , Staphylococcus aureus ou encore des agents pathogènes opportunistes.

Les compositions selon la présente invention peuvent être utilisées également pour lutter contre les virus et les infections favorisées par eux. Ceci est valable en particulier pour les virus de la grippe et les virus HIV.

La présente invention a en outre pour objet des anticorps monoclonaux ou polyclonaux dirigés contre un épitope des protéines selon l'invention.

La présente invention a également pour objet des hybridomes produisant des anticorps monoclonaux dirigés contre un épitope des protéines selon l'invention.

La présente invention a également pour objet un procédé de dosage ou de détection de la protéine native dans des tissus et des liquides biologiques qui comprend l'utilisation d'anticorps monoclonaux ou polyclonaux selon l'invention. A cet effet on peut utiliser notamment une méthode de type ELISA.

On décrira ci-après plus en détail les conditions permettant d'obtenir les produits selon l'invention, les caractéristiques de ces produits, ainsi que les propriétés des produits selon l'invention.

I - Obtention et caractérisation des produits selon l'invention.

Lors de l'obtention des produits selon l'invention, l'activité biologique des produits obtenus a été appréciée par leur capacité, comme dans le cas de la protéine MGP, après leur injection à des souris, à protéger ces dernières contre une infection létale à Listeria monocytogenes . Pour cela, les différentes fractions sont, après lyophilisation, reprises par du sérum physiologique non pyrogène et injectée à des doses variables en intraveineuse à des souris OF1 femelles, âgées de 6 à 8 semaines. Vingt-quatre heures plus tard, ces souris reçoivent, par voie veineuse une dose létale de Listeria monocytogenes (100.000). Les résultats ont été le plus souvent appréciés par la survie des animaux, 4 jours après l'infection. Dans d'autres expériences, l'activité fut quantifiée après numération bactérienne dans les rates des souris, 48 heures après l'infection.

1) Collecte, conservation et traitement des urines :

Les urines de 24 heures provenant de personnes des deux sexes sont gardées à 4°C entre chaque miction, dans des flacons stériles. Elles sont ensuite congelées et gardées à - 20°C jusqu'à leur utilisation. Ultérieurement, après décongélation, elles sont centrifugées et le surnageant obtenu est concentré 50 fois par ultrafiltration sur une membrane d'ultrafiltration Amicon YM10. Au concentrat ainsi obtenu est ajoutée une solution de phosphate de sodium 0,7M afin d'obtenir une concentration finale en phosphate de 0.07M pH 6.8.

2) Chromatographie sur une colonne de DEAE Affigel[R] blue :

Le concentrat urinaire (20 ml pour un litre d'urine) est déposé sur une colonne contenant 10 ml de gel de DEAE Affigel[R] blue au préalable équilibré avec le même tampon que précédemment. La colonne est ensuite intensivement lavée avec le tampon jusqu'à ce que la densité optique (280 nm) de l'éluat soit inférieure à 0,05. L'élution des protéines retenues parmi lesquelles est présente celle douée d'activité immunostimulante est effectuée par le même tampon contenant du chlorure de sodium 0,2 M. La fraction correspondant au pic de cet éluat est alors concentrée sur Amicon PM 30 et dialysée contre de l'eau distillée pyrolysée. Au dialysat est ajouté du bicarbonate d'ammonium 1M de façon à obtenir une concentration finale en bicarbonate d'ammonium de 0,1 M.

3) Hydrolyse par la pronase.

A la solution dialysée ci-dessus, contenant 2 mg de protéine par ml, est ajouté, 1% en poids de pronase (Sigma). Après incubation du mélange pendant 9 heures à 37°C, on ajoute à nouveau 1% de pronase. L'incubation est poursuivie à 37°C pendant 15 heures. La solution est alors lyophilisée. Le lyophilisat est dilué en tampon phosphate de sodium 0,1 M pH 7,2 à une concentration protéique de 20 unités de densité optique (280 nm).

4) Filtration sur tamis moléculaire :

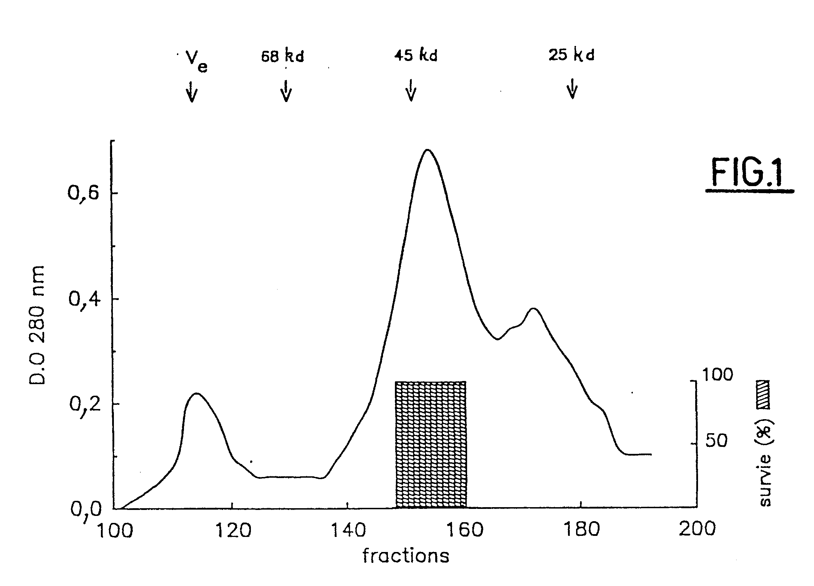

Cette solution est déposée sur une colonne de perméation AcA 54 (IBF) équilibrée en tampon phosphate 0,1M pH 7,2 avec un débit de 8 ml/heure. On a reporté sur la Fig. 1 les valeurs de densité optique à 280 nm des différentes fractions ainsi que les points de passage de marqueurs de masse moléculaire connue (68 kD = albumine bovine, 45 kD = ovalbumine, 25 kD = chymotrypsinogène). Ces valeurs sont indiquées à ± 5 %. On a également reporté sur la Fig. 1 la zone qui correspond à 100% de survie dans le test de l'infection à Listeria monocytogenes . On constate que toute l'activité immunostimulante est retrouvée dans une zone de masse moléculaire comprise entre 40 et 50 kD (Fig. 1). Ceci constitue la fraction active.

5) Chromatographie sur une colonne d'immunoaffinité :

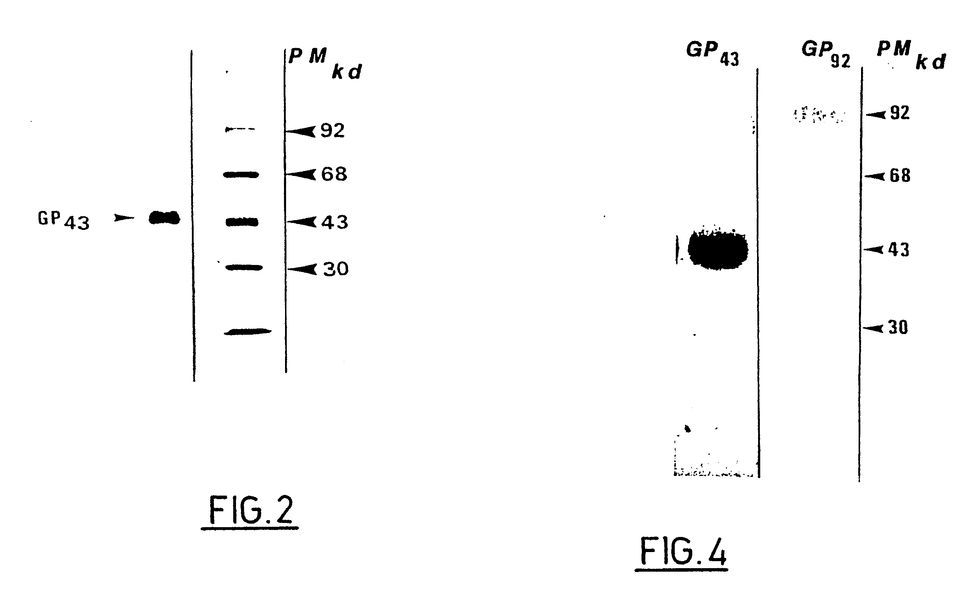

Cette fraction active, après dialyse contre de l'eau distillée pyrolysée et lyophilisation, est reprise par un tampon phosphate de sodium 10 mM pH 7,2 contenant 0.15M de chlorure de sodium. Puis elle est déposée sur une colonne de Sépharose 4B activée au bromure de cyanogène (PHARMACIA) sur laquelle ont été greffés des anticorps polyclonaux monospécifiques dirigés contre l'$\alpha_1$ glycoprotéine acide à raison de 1 mg d'anticorps par ml de gel. Le débit de la colonne est de 8 ml/heure. Les fractions contenant la protéine immunostimulante sont dialysées contre de l'eau distillée pyrolysée puis lyophilisées. L'examen par électrophorèse en gel SDS à 1%, de ce lyophilisat montre une seule bande après coloration au bleu de Coomassie ou au nitrate d'argent. On a représenté sur la Fig. 2 le résultat de l'électrophorèse. Pour déterminer le poids moléculaire de la molécule active, on a utilisé les marqueurs de poids moléculaire de référence suivants :
92 kD (phosphorylase b), 68 kD (albumine bovine), 43 kD (ovalbumine), 30 kD (anhydrase carbonique). Ces valeurs sont indiquées à ± 5 %.

La protéine ainsi obtenue est glycosylée. En effet, après hydrolyse de cette protéine par de l'acide sulfurique 6N et dosage des sucres par l'anthrone selon la technique de MELVIN-SCOTT, le pourcentage en sucre est d'environ 10%.

Ces résultats amènent donc à considérer que cette protéine est une glycoprotéine de poids moléculaire de 43 kD (± 5 %). Cette glycoprotéine est par suite appelée GP 43.

Cette glycoprotéine est résistante à l'hydrolyse par les enzymes suivants : 1) trypsine, 2) protéase de staphylocoque V8, 3) pronase.

La quantité de GP 43 obtenue par le procédé décrit ci-dessus, à partir d'urines d'hommes et de femmes cliniquement normaux est de 0,2-0,5 $\mu$g/ml.

II - Obtention d'anticorps monoclonaux :

Chez des souris Balb/c femelles agées de 6 à 8 semaines fut injectée une fraction semi-purifiée de GP 43 dans de l'adjuvant de Freund complet à raison de 125 $\mu$g par souris en cinq endroits différents, par voie sous cutanée, dans le dos de la souris. Deux semaines plus tard, les souris reçoivent, par la même voie, 125 $\mu$g de la même fraction diluée dans une solution de chlorure de sodium 0,15 M. Lorsque par test ELISA, fut constaté un taux d'anticorps au 1/1000, une injection de rappel fut pratiquée dans les mêmes conditions que précédemment. Cinq jours plus tard, les cellules spléniques furent isolées, fusionnées avec des cellules de myélome SP2/0 Ag 14 dans un rapport de 10 à 1 en présence de polyéthylène glycol 1000. Les cellules furent réparties dans des boîtes de culture à 24 godets à raison de 1.000.000 par godets en présence de 10% de sérum de cheval. Les hybridomes positifs obtenus ont été clonés deux fois par dilution limite dans des plaques de microtitration à 96 puits en utilisant un tapis de macrophages comme adjuvant de la croissance des hybridomes. Ainsi fut sélectionné un anticorps monoclonal appelé K5 2,5 G2 qui réagit en test ELISA avec la fraction antigénique de départ et avec des antigènes décrits ci-dessous. Il reconnaît uniquement la protéine de 92 kD après transfert des protéines 43 kD et 92 kD sur feuille de nitrocellulose. Mais il reconnaît ces molécules en test ELISA. L'hybridome K5.2.5 G2 a été déposé le 24.7.89 à la CNCM sous le n° I 898.

III - Obtention d'anticorps polyclonaux :

Des lapins néozélandais ont été injecté par voie sous-cutannée dans le coussinet plantaire avec 100 $\mu$g de GP 43 homogénéisée dans de l'adjuvant complet de Freund. Deux semaines plus tard, les lapins reçoivent par voie sous-cutanée dans la région dorsale 100 $\mu$g de GP 43 en solution dans 2 ml de chlorure de sodium 0,15 M et ceci, tous les quinze jours. Des anticorps anti $\alpha_1$-glycoprotéine acide pouvant persister, ils sont éliminés par passage sur une colonne d'immunoaffinité greffée avec de l'$\alpha_1$-glycoprotéine acide à raison de 1 mg d'$\alpha_1$-glycoprotéine acide par ml de gel. La présence d'anticorps reconnaissant GP 43 a été établie par test ELISA.

## IV - Recherche de la protéine native :

L'utilisation de pronase pour l'obtention de GP 43 nous a amenés à considérer que cette protéine est le produit d'hydrolyse d'une protéine de poids moléculaire plus important laquelle a été recherchée dans l'éluat obtenu après chromatographie sur bleu de cibacron (I.3).

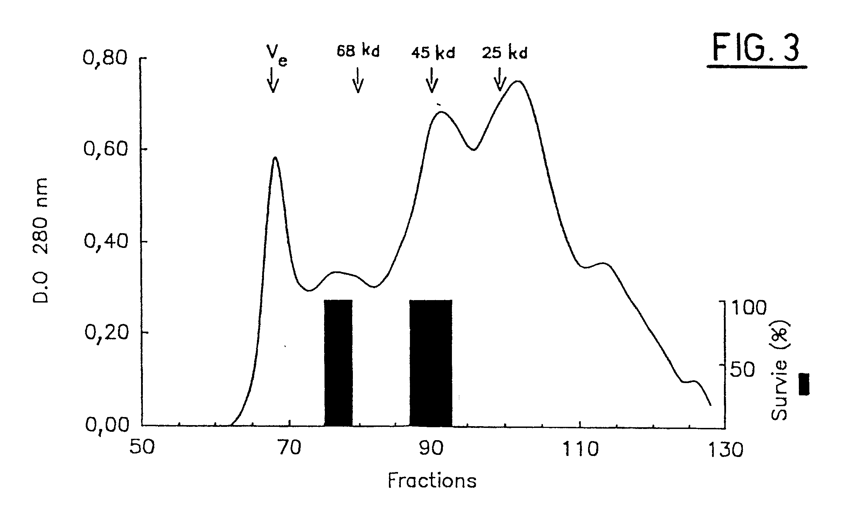

Pour cela, l'éluat est chromatographié sur un gel de perméation AcA 44 (IBF) équilibré en tampon phosphate de sodium 0,1 M pH 7,2; le débit étant de 10 ml/heure. Ainsi que le montre la Fig. 3, sur laquelle on a reporté la densité optique à 280 nm et le taux de survie pour les différentes fractions, l'activité immunostimulante est retrouvée dans deux pics ayant des poids moléculaires apparents de 90-95 kD et 40-45 kD. Pour déterminer le poids moléculaire des molécules actives, on a utilisé les marqueurs de poids moléculaire de référence suivants : 68 kD (albumine bovine), 45 kD (ovalbumine) et 25 kD (chymotrypsinogène). Ces valeurs sont indiquées à ± 5 %.

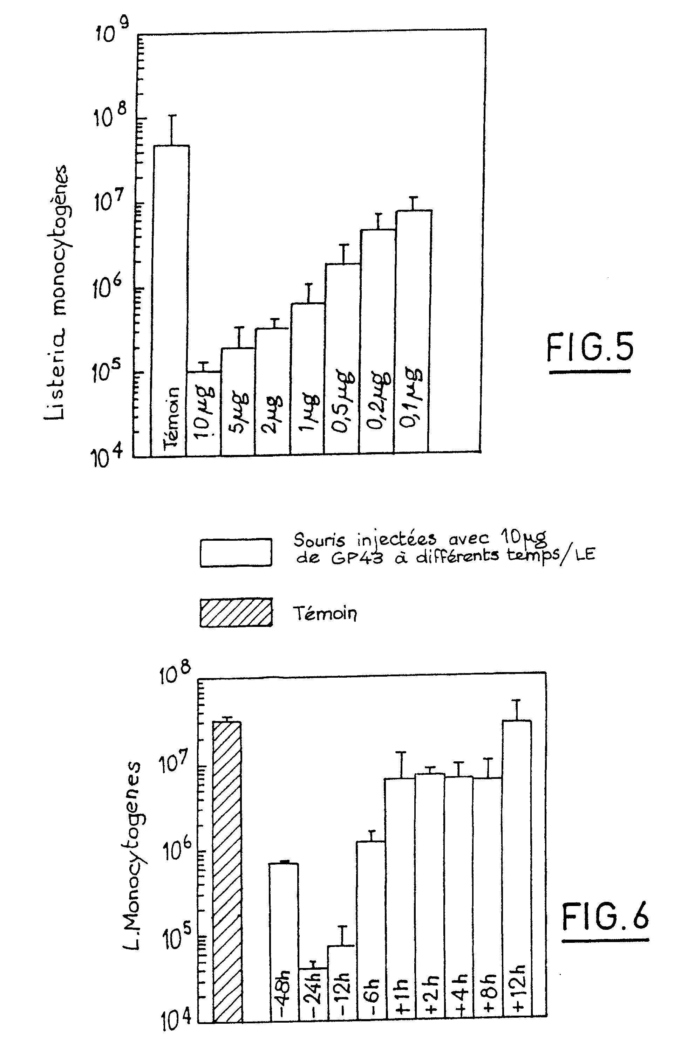

Après électrophorèse dans un gel de polyacrylamide SDS à 1%, sans réduction préalable et transfert des protéines de PM 90-95 kD sur une feuille de nitrocellulose, les anticorps polyclonal et monoclonal ne reconnaissent qu'une seule protéine de PM 92 kD. On a représenté sur la Fig. 4 le résultat de l'électrophorèse de la protéine native ainsi que de la protéine GP 43.

Lorsque l'électrophorèse est réalisée en présence de réducteur (dithiothreitol 20 mM), il est observé une disparition de la double bande 92 kD (± 5 %) au profit d'une bande de 45 kD (± 5 %).

La protéine de 45 kD est reconnue par les anticorps polyclonal et monoconal en test ELISA et en Western Blot.

L'action de la pronase sur la protéine de 92 kD et sur la protéine de 45 kD aboutit à l'apparition d'une protéine de 43 kD (± 5 %).

Ces résultats permettent de penser que la protéine de 43 kD résulte de la disparition des ponts disulfure intercaténaire présents sur la région éliminée après action de la pronase.

## V - Recherche de GP 43 dans le sérum humain :

L'acidité de cette glycoprotéine (pI : 3,3) et son poids moléculaire nous ont amenés à tester divers antisérums dirigés contre des protéines ayant les mêmes caractéristiques physicochimiques :
- $\alpha_1$ glycoprotéine acide
- $\alpha_1$ antitrypsine
- Gc globuline

Il ne fut pas trouvé de communauté antigénique avec GP 43.

De plus, l'injection à des souris de 5 à 20 $\mu$g d'une préparation obtenue selon les procédés décrits dans la littérature (Schutze et al.) d'$\alpha_1$-glycoprotéine acide n'est suivie d'aucun effet protecteur contre une infection par Listeria monocytogenes .

Les anticorps monoclonaux et polyclonaux peuvent être avantageusement utilisés dans un coffret de diagnostic afin de mettre en évidence et d'autoriser le dosage quantitatif des antigènes 92 kD et 43 kD selon les techniques connues de l'homme de l'art.

## VI - Activité biologique de GP 43 :

1) protection contre une infection murine par Listeria monocytogenes .

Des groupes de quatre souris OF1 femelles âgées de 6 à 8 semaines, reçoivent par voie veineuse: soit du sérum physiologique (groupe témoin) soit 0,1, 0,2, 0,5, 1, 2, 5 et 10 $\mu$g de GP 43. Vingt-quatre heures plus tard, les souris de tous ces groupes sont infectées par voie veineuse avec un inoculum de 100.000 Listeria monocytogenes . Quarante-huit heures plus tard, des numérations bactériennes sont pratiquées dans les rates.

On a reporté sur la Fig. 5 le $\log_{10}$ du nombre de Listeria monocytogenes pour des doses de 0,1 à 10 microgrammes. Il apparaît que l'injection de 0,1 $\mu$g de GP 43 entraîne une diminution d'un facteur 6 du nombre des bactéries spléniques. L'effet maximal est obtenu avec 10 $\mu$g de protéine GP 43.

2) Des groupes de quatre souris OF1 femelles âgées de 6 à 8 semaines, reçoivent par voie veineuse soit du sérum physiologique (témoins), soit 10 $\mu$g de GP43, 48 h, 24 h et 6 h avant un inoculum intra veineux de 100.000 Listeria monocytogenes ou 1 h, 2 h, 4 h, 8 h et 12 h après cet inoculum. Quarante-huit heures après l'infection les numérations bactériennes sont pratiquées dans les rates de toutes les souris. Ainsi qu'il apparaît dans la Fig. 6, une réduction du nombre de bactéries n'apparaît que lorsque la protéine GP 43 est injectée 48 heures avant l'infection et est maximale quand l'injection a lieu 24 heures avant l'infection. Cette réduction de nombre des bactéries est encore notable lorsque GP 43 est injecté 1 à 8 heures après l'infection.

3) Activité cytostatique de cellules péritonéales envers les cellules de la tumeur de Lewis.

Des cellules péritonéales de souris C57 Bl 6 (1.000.000 par tube) sont incubées avec 0,01, 0,02, 0,05, 0,1, 0,2, 0,5, 1 et 2 $\mu$g/ml de GP 43 pendant 48 heures. Les cellules de Lewis sont ajoutées à raison de 50.000 par tube et, vingt-quatre heures plus tard, l'incubation est poursuivie en présence de déoxyuridine marquée à l'iode 125. L'incorporation de l'isotope dans les cellules de la tumeur de Lewis est mesurée 17 heures plus tard.

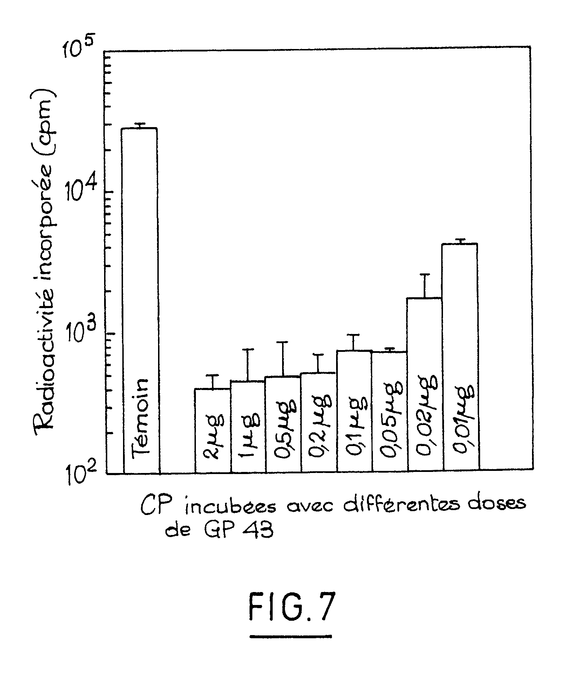

On a reporté sur la Fig. 7 le nombre de coups par minute pour les différents échantillons : CP (cellules peritonéales), CP avec les concentrations ci-dessus mentionnées de GP 43.

Ainsi qu'il apparaît sur cette figure, on note une diminution de l'incorporation de déoxyuridine d'un facteur allant de 7 à 70 pour des concentrations de GP43 allant de 0,01 μg à 2 μg/ml.

4) Activité bactéricide des macrophages de souris envers Mycobacterium tubertulosis (BCG).

L'incubation des macrophages avec 5 μg/ml de GP 43 entraîne une diminution de l'incorporation d'uracile tritiée dans les mycobactéries.

5) Activité Leishmanicide des macrophages de souris C₅₇ Bl/6.

L'incubation de macrophages dérivés de la moelle osseuse de souris C₅₇ Bl/6 avec 2 à 5 μg/ml de GP 43 permet de constater :

- un plus grand étalement de ceux-ci
- une restriction de la multiplication intracellulaire de Leishmanies.


VII - Hydrolyse de la protéine Gp43 par le bromure de cyanogène

On dissout 20 μg de GP43 dans 200 μl d'une solution contenant 10 mg de bromure de cyanogène (Merck) dans 1 ml d'acide formique à 70%. On incube ce mélange 24 heures dans l'obscurité à la température de la pièce. On évapore le mélange par centrifugation sous vide. On dissout le résidu obtenu dans 100 μl d'acide trifluoroacétique (TFA). On incube 30 minutes à 37°C. On dilue 10 fois avec de l'eau pyrolysée et injecte immédiatement sur colonne d'HPLC C₄ en phase reverse avec les solvants suivants :
Solvant A : 0,1% TFA
Solvant B : 0,1% TFA + 70% acétonitrile.

On élue de 0 à 5 minutes avec le solvant A et, de 0 à 75 minutes, on élue avec un gradient linéaire 100% de A au début pour terminer avec 100% de B.

On récupère ainsi deux peptides dont le séquençage montre qu'ils ont la structure suivante :
- peptide 1 : Met-Ala-Glu-Thr-Glu-Val-Pro-Phe-Leu-Arg-Asp-Asn
- peptide 2 : Met-Ser-Leu-Val-Ile-Pro-Gly-Val

Le mélange des peptides ainsi obtenu a conservé l'activité immunostimulante anti Listeria , évaluée selon les mêmes conditions expérimentales que celles décrites au point I.


**Revendications**

1. Glycoprotéine immunostimulante ayant les caractéristiques suivantes :

a. elle a une masse moléculaire d'environ 43 kD

b. elle résiste à l'hydrolyse par la trypsine et par la pronase

c. elle présente un pourcentage en sucre d'environ 10%

d. elle peut être obtenue à partir d'urine humaine

e. elle comprend les 2 séquences peptidiques suivantes :
- peptide 1 : Met-Ala-Glu-Thr-Glu-Val-Pro-Phe-Leu-Arg-Asp-Asn
- peptide 2 : Met-Ser-Leu-Val-Ile-Pro-Gly-Val.

2. Glycoprotéine selon la revendication 1, caractérisée en ce qu'elle est reconnue dans un test ELISA par l'anticorps monoclonal K5.2.5 G₂ produit par l'hybridome K.5.2.5.G2 déposé à la CNCM sous le numéro I 898.

3. Peptide de formule Met-Ala-Glu-Thr-Glu-Val-Pro-Phe-Leu-Arg-Asp-Asn.

4. Peptide de formule Met-Ser-Leu-Val-Ile-Pro-Gly-Val

5. Protéine native ayant une masse moléculaire d'environ 92 kD qui présente une activité immunostimulante et qui donne la glycoprotéine selon la revendication 1 par hydrolyse par la pronase.

6. Procédé d'obtention d'une protéine selon la revendication 5 qui consiste à séparer cette protéine de l'urine humaine.

7. Procédé selon la revendication 6, caractérisé par les étapes suivantes :
- collecte, conservation et traitement des urines
- chromatographie sur une colonne de DEAE Affigelᴿ Blue.

8. Procédé d'obtention d'une protéine selon la revendication 1, caractérisé en ce qu'il consiste à hydrolyser par la pronase une protéine selon la revendication 5.

9. Procédé selon la revendication 8, caractérisé par les étapes suivantes :
- hydrolyse par la pronase d'une protéine selon la revendication 4
- filtration sur tamis moléculaire
- chromatographie sur colonne d'immunoaffinité anti-α₁-glycoprotéine acide.

10. Anticorps monoclonaux et polyclonaux dirigés contre une protéine ou un peptide selon l'une quelconque des revendications 1 à 5.

11. Hybridomes caractérisés en ce qu'ils produisent des anticorps monoclonaux selon la revendication 10.

12. Composition thérapeutique comprenant une protéine ou un peptide selon l'une quelconque des revendications 1 à 5.

13. Procédé de dosage ou de détection d'une protéine selon la revendication 5 dans des tissus et liquides biologiques, comprenant l'utilisation d'anticorps monoclonaux ou polyclonaux dirigés contre un épitope de cette protéine.

FIG.1

EP 0 419 298 A1

GP₄₃ ►

PM kd

◄ 92

◄ 68

◄ 43

◄ 30

FIG.2

GP₄₃   GP₉₂   PM kd

◄ 92

◄ 68

◄ 43

◄ 30

FIG.4

FIG. 3

FIG.5

Souris injectées avec 10µg
de GP43 à différents temps/LE

Témoin

FIG.6

FIG.7

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.5 ) |
|---|---|---|---|
| Y,D | Medline Abstarct Nb 88297893<br>FONTAN E et al: " Inflammation and anti-tumor resistance. IV. Induction of cytostatic activity of murine peritoneal cells by a mouse granuloma protein." & INT.J.CANCER, 15/8/1988, Vol 42, No 2 p 267-72. | 1-13 | A61K45/05<br>A61K35/22 |
| Y | Medline Abstarct Nb 86321788<br>FONTAN E et al: "Increased phagocytic activity in mice treated by a mouse granuloma protein." & ANN.INST.PASTEUR.IMMUNOL., 7-8/1986, Vol 137D, No 1, p 93-107. | 1-13 | |
| Y,D | Medline Abstract Nb 84016059<br>FONTAN E et al: "Immunostimulatory mouse granuloma protein." & PROC.NATL.ACAD.SCI.USA., 10/1983, Vol 80, No 20 p 6395-98. | 1-13 | |

| | DOMAINES TECHNIQUES RECHERCHES (Int. Cl.5 ) |
|---|---|
| | A61K<br>C07K |

Le présent rapport a été établi pour toutes les revendications

| Lieu de la recherche | Date d'achèvement de la recherche | Examinateur |
|---|---|---|
| BERLIN | 18 OCTOBRE 1990 | AVEDIKIAN P.F. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons
& : membre de la même famille, document correspondant

EPO FORM 1503 03.82 (P0402)